# EUROPEAN PATENT SPECIFICATION

(11) **EP 2 674 158 B1**
(45) Date of publication and mention of the grant of the patent: **29.07.2015**
(21) Application number: 12172042.9
(22) Date of filing: 14.06.2012
(51) Int. Cl.: A61K 31/343, A61K 9/00, A61K 9/06

(54) **Treatment and prevention of cardiac arrhythmias**
Behandlung und Prävention von Herzrhythmusstörungen
Traitement et prévention d'arythmies cardiaques

(43) Date of publication of application: 18.12.2013
(73) Proprietor: Szegedi Tudományegyetem, 6720 Szeged (HU)
(72) Inventor: Varró, András, 6726 Szeged (HU); Mátyus, Péter, 1032 Budapest (HU); Baczkó, István, 6723 Szeged (HU); Falkay, György, 6725 Szeged (HU); Jost, Norbert, 6724 Szeged (HU); Leprán, István, 6725 Szeged (HU); Sztojkov-Ivanov, Anita, 5900 Orosháza (HU); Virág, László, 6725 Szeged (HU); Buzás, Norbert, 6750 Algyõ (HU)
(74) Representative: Lengyel, Zsolt

(56) References cited:
- ROBERT G. TIELEMAN ET AL: "Efficacy, Safety, and Determinants of Conversion of Atrial Fibrillation and Flutter With Oral Amiodarone", THE AMERICAN JOURNAL OF CARDIOLOGY, vol. 79, no. 1, 1 January 1997 (1997-01-01), pages 53-57, XP55039379, ISSN: 0002-9149, DOI: 10.1016/S0002-9149(96)00675-3
- BOLDERMAN R W ET AL: "Atrium-targeted drug delivery through an amiodarone-eluting bilayered patch", JOURNAL OF THORACIC AND CARDIOVASCULAR SURGERY, MOSBY-YEAR BOOK, INC., ST. LOUIS, MO, US, vol. 140, no. 4, 1 October 2010 (2010-10-01), pages 904-910, XP027294419, ISSN: 0022-5223 [retrieved on 2010-09-16]
- ZHOU LI ET AL: "A comparison of the antifibrillatory effects of desethylamiodarone to amiodarone in a swine model", JOURNAL OF CARDIOVASCULAR PHARMACOLOGY, RAVEN PRESS, NEW YORK, NY, vol. 34, no. 3, 1 January 1999 (1999-01-01), pages 440-445, XP008156842, ISSN: 0160-2446, DOI: 10.1097/00005344-199909000-00019
- STARK G ET AL: "Comparison of acute electrophysiological effects of amiodarone and its metabolite desethylamiodarone in Langendorff perfused guinea pig hearts", BASIC RESEARCH IN CARDIOLOGY, STEINKOPFF, DARMSTADT, DE, vol. 86, no. 2, 1 March 1991 (1991-03-01), pages 136-147, XP008156880, ISSN: 0300-8428
- JAHANAVI KHARIDIA ET AL: "EFFECTS OF DESETHYLAMIODARONE ON THE ELECTROCARDIOGRAM IN CONSCIOUS FREELY MOVING ANIMALS: PHARMACOKINETIC AND PHARMACODYNAMIC MODELING USING COMPUTER-ASSISTED RADIO TELEMETRY", BIOPHARMACEUTICS & DRUG DISPOSITION, vol. 17, no. 2, 1 March 1996 (1996-03-01), pages 93-106, XP055104039, ISSN: 0142-2782, DOI: 10.1002/(SICI)1099-081X(199603)17:2<93::AI D-BDD930>3.0.CO;2-I
- Guang-Fei Shi ET AL: "Mechanism of the delayed onset of action after intravenous amiodarone for treatment of ventricular arrhythmias", Drug development research, vol. 58, no. 1, 1 January 2003 (2003-01-01), pages 145-148, XP55039416, ISSN: 0272-4391, DOI: 10.1002/ddr.10144

## Description

The invention relates to a compound selected from the group consisting of desethylamiodarone and pharmaceutically acceptable salts, hydrates and solvates thereof, as well as pharmaceutical composition comprising the compound together with a pharmaceutically acceptable excipient, vehicle or carrier, for use in the treatment and prevention of atrial fibrillation.

Cardiovascular diseases including sudden cardiac death and stroke are among the leading causes of mortality in industrialized countries. The most serious ventricular arrhythmia - ventricular fibrillation (VF) - causes more than 300 000 deaths in the USA annually. Atrial fibrillation (AF) is one of the most common arrhythmia entities with 2-5 % incidence in the elderly (60-65 years) population. In addition, AF often elicits dangerous or life threatening ventricular arrhythmias including VF and also contributes to the pathogenesis of stroke. At present the pharmacological treatment of arrhythmias including AF is not satisfactory, since the available drugs either do not control arrhythmias properly or induce serious side effects. Therefore, there is an increasing demand for safe and effective new drugs to treat AF and arrhythmias in general.

Chronic amiodarone (AMIO) application is the most effective pharmacological treatment to combat AF and arrhythmias with less proarrhythmic risk than other currently used antiarrhythmics (Shinagawa et al, 2003; Ravens, 2010). However, AMIO - which has a very complex mode of action inhibiting cardiac sodium, calcium, potassium currents and beta adrenoceptors - also exerts serious extracardiac adverse effects like pulmonary fibrosis, hepatotoxicity, photodermatosis, cornea deposits etc. which greatly limit its clinical use (Tisdale et al, 1995). The toxic effect of AMIO is favoured by its slow elimination (plasma half life: 40-80 days!) resulting in drug accumulation in different tissues of the body. It is known that during chronic AMIO treatment an electrophysiologically active amiodarone metabolite, desethylamiodarone (DEA) appears in the plasma and tissues including the heart (Flanagan et al, 1982; Nattel et al, 1986). Since both AMIO and DEA contain iodine it is likely that they inhibit and interfere (Shi et al, 2008; van Beeren et al, 1995; van Beeren et al, 1999; Latham et al, 1987) with cardiac thyroid receptors and exert their antiarrhythmic effect partly by this mechanism. It was reported earlier that DEA binds to cardiac thyroid receptors with higher affinity (van Beeren et al, 1995; Latham et al, 1987) than AMIO.

It is known from previously published works that DEA after single acute application has similar cardiac electrophysiological and ventricular antiarrhythmic effects as AMIO (Nattel et al, 1986; Talajic et al, 1987; Varró et al, 1987; Nattel et al, 1988).

Tieleman et al. (1997) studied the chronic application of amiodarone (AMIO) in patients buffering from atrial fibrillation or flutter who were refractory to other conventional antiarrhythmic drugs.

Bolderman et al. (2010) investigated the effect of AMIO by local epicardial application against postoperative atrial arrhythmias.

Zhou et al. (1999) investigated the effect of AMIO and DEA on ventricular arrhythmias by intravenous administration. Stark et al. (1991) examined the electrophysiological effects of AMIO and DEA on perfused hearts.

Kharida et al. (1996) disclosed experiments confirming the facts already known, i.e. that DEA as the active metabolite of AMIO contributes to the efficacy of the latter.

Guang-Fei et al. (2003) studies the action of intravenous AMIO.

It is clear that there is long standing need for a safer and effective treatment of atrial fibrillation.

The present inventors surprisingly found that chronic administration of DEA can be used to prevent and/or abolish atrial fibrillation (AF). The prior art did not disclose that chronic DEA treatment would be useful for AF; the closest finding in the state of the art can be considered the study by Kato (1998), showing that chronic DEA administration elicited similar electrophysiological action compared to its parent compound AMIO in rabbit atria. However, this finding has no real relevance on the present invention, since the cardiac action potential in rabbits is controlled by distinctly different transmembrane ion channels compared to those in dogs and humans (Wang et al, 1995; Wang et al, 1999), therefore the person skilled in the art would not have reasonable expectation of success to simply follow on these results and arrive at the present invention. In addition, this study did not report or suggest that this similar electrophysiological behavior would lead to any significant chronic effect on cardiac arrhythmias, including AF which is the essential feature of the present invention. On the other end, the person skilled in the art could not base his attempt to create the present invention on the prior art regarding the acute effects of DEA, since it has only been reported in ventricular arrhythmias but not in AF (Zhou et al, 1998).

Accordingly, the present invention provides a compound selected from the group consisting of desethylamiodarone and pharmaceutically acceptable salts, hydrates and solvates thereof,, together with a pharmaceutically acceptable excipient, vehicle and/or carrier for use in the treatment and/or prevention of atrial fibrillation by oral adminisiration.

In a further specific embodiment, the compound or composition of the invention is administered orally, sublingually, buccally, or parenterally.

In another specific embodiment, the compound or composition of the invention is administered chronically.

In another specific embodiment, the compound or composition is administered once a day.

### Detailed description

In the present invention, we present novel, previously not available and not published data on the effects of both acute and chronic administration of DEA:
As a preliminary finding, we established that acute application of 5 µM DEA resulted in a similar protective effect against AF compared to that of 10 µM AMIO in isolated rabbit cardiac atrial preparations. Although the prior art studied the acute effects of DEA, there is no disclosure for effecting atrial fibrillation.

Chronic, 3-week oral (25 mg/kg/day) DEA treatment resulted in similar cardiac tissue concentration and antiarrhythmic action as chronic AMIO treatment in double dose (50 mg/kg/day) in conscious rats after coronary artery ligation. Again, the prior art did not teach the chronic application of DEA.

Chronic, 3-week oral (25 mg/kg/day) DEA treatment resulted in similar cardiac tissue concentration and protective antiarrhytmic effects to that measured following the higher 50 mg/kg/day oral AMIO treatment in the chronic atrial tachypacing induced AF model in dogs. In these dogs, the liver and lung tissue concentrations of DEA were more than three times higher in the chronic AMIO treated dogs compared to animals receiving chronic DEA treatment. These results are in good agreement with results showing DEA accumulation in human alveolar epithelium-derived cell lines following AMIO treatment (Seki et al., 2008). Based on this observation it can be concluded that chronic AMIO treatment would greatly enhance the risk for hepato- and pulmonary toxic complications compared to treatment with DEA alone.

Chronic treatment of uninstrumented dogs with 30 mg/kg desethylamiodarone resulted in similar antiarrhythmic cellular electrophysiological changes in cardiac atrial and ventricular tissue to 45 mg/kg chronic amiodarone treatment. The tissue levels for desethylamiodarone both in the cardiac atrial and ventricular tissue were similar. The same observation was made for liver, lung and kidney tissue levels which can be important for possible organ toxicity issues. It is important to emphasize that during chronic amiodarone treatment in addition to the metabolite (desethylamiodarone) deposition even higher tissue amiodarone depositions were observed in the heart, lung, liver and the kidney. In rats, chronic 28-day oral DEA treatment (100 mg/kg/day) resulted in reduced pulmonary- and hepatotoxicity than 200 mg/kg/day AMIO treatment. In addition, in this study the elimination of DEA was significantly faster than that of AMIO.

Accordingly, the facts presented above and discussed in more detail below and in the experimental section, chronic DEA treatment can be advantageously used to prevent and/or abolish atrial fibrillation (AF). In particular, DEA administration at half of the dose than that of AMIO results in similar cardiac tissue DEA levels and has similar protective effect in AF than its parent compound AMIO. If patients are treated with the metabolite - i.e. with DEA - we can eliminate the parent compound AMIO from different other types of tissues. This should be advantageous since AMIO can contribute to various organ toxicities which is not the case if treatment is carried out directly with DEA only. According to the present invention, the elimination of DEA is faster than that of AMIO. In addition, the elimination of DEA is faster if AMIO is not present in the tissues.

It is suggested that during chronic AMIO treatment the majority of therapeutically useful effects related primarily to DEA and the presence of relatively high concentration of AMIO in different tissues is not necessary for the therapeutically useful action but only contributes to the serious side effects observed during chronic AMIO treatments. Therefore, by substituting chronic AMIO treatment with chronic DEA treatment a still sufficiently strong antiarrhythmic effect is achieved with significantly less adverse effects. In addition, since AMIO treatment often causes interactions with other drugs such as digitalis, statins, warfarin etc, chronic DEA treatment would also limit these possible drug interactions. The first step of degradation of AMIO and DEA takes place via the same and the next steps via different enzyme systems which would also favour DEA treatment over its parent compound AMIO.

Our present new and previously not published results suggest that chronic oral treatment with DEA resulted in similar cardiac tissue levels compared to that of chronic AMIO treatment and showed an equivalent degree of antiarrhythmic effect against coronary artery ligation induced ventricular arrhythmias in rats. This is an important factor since AF often initiates ventricular arrhythmias, including VF, which should be also treated or prevented as effectively as possible.

Therefore, in summary it can be expected that chronic DEA treatment would be more or at least similarly effective than chronic AMIO treatment with better pharmacokinetics, and very importantly, with fewer adverse effects and with reduced unexpected drug interactions.

Accordingly, the present invention provides a compound selected from the group consisting of desethylamiodarone according to formula (I), didesethylamiodarone according to formula (II), and pharmaceutically acceptable salts, hydrates and solvates thereof, for use in the treatment and prevention of atrial fibrillation. DEA, [(2-Butylbenzofuran-3-yl)-[4-(2-ethylaminoethoxy)-3,5-diiodophenyl]methanone; C23H25I2NO3; CAS Registry Number: 83409-32-9] is a metabolite of amiodarone, having the following chemical structure: 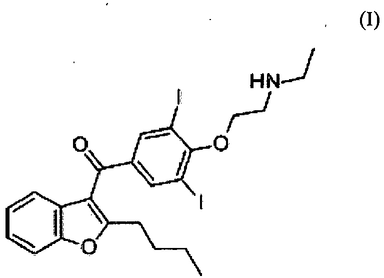 diDEA [(di-N-desethylamiodarone; [4-(2-Aminoethoxy)-3,5-diiodophenyl](2-butyl-3-benzofuranyl)methanone; C21H21I2NO3; CAS Registry Number: 94317-95-0] is another metabolite of amiodarone having the following chemical structure:

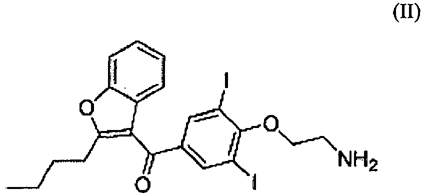

The term compound as used herein means compounds, or a compound, of formula (I) and includes all polymorphs and crystal habits thereof, prodrugs and isomers thereof (including optical, geometric and tautomeric isomers), and mixtures thereof.

The person skilled in the art will appreciate that the compound of formula (I) can be present in the form of pharmaceutically acceptable salts, for example, non-toxic acid addition salts formed with inorganic acids such as hydrochloric, hydrobromic, sulphuric and phosphoric acid, perchlorate, with organo-carboxylic acids, or with organo-sulphonic acids. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, rotate, oxalate, palmitate, pamoate, phosphate/hydrogen phosphate/dihydrogen phosphate, saccharate, stearate, succinate, tartrate, tosylate, adipate, cyclamate, tannate, pyroglutamate, xinafoate (1-hydroxynaphthalene-2-carboxylate) and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, Weinheim, Germany, 2002).

The compound of formula (I) may exist in both unsolvated and solvated forms. The term "solvate" is used herein to describe a molecular complex comprising the compound and a stoichiometric amount of one or more pharmaceutically acceptable solvent molecules, for example, ethanol. The term "hydrate" designates a complex wherein the solvent is water.

In the specification, all references to the compound of formula (I) include references to salts, solvates, hydrates and complexes thereof and to solvates and complexes of salts thereof.

In specific circumstances, so-called 'pro-drugs' of the compound of formula (I) are also within the scope of the invention. Thus certain derivatives of the compound of formula (I) which may have little or no pharmacological activity themselves can, when administered into or onto the body, be converted into compounds of formula (I) having the desired activity, for example, by hydrolytic cleavage. Such derivatives are referred to as 'prodrugs'. Further information on the use of prodrugs may be found in Pro-drugs as Novel Delivery System, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs in accordance with the invention can, for example, be produced by replacing appropriate functionalities present in the compound of formula (I) with certain moieties known to those skilled in the art as 'pro-moieties' as described, for example, in Design of Prodrugs by H. Bundgaard (Elsevier, 1985).

Some examples of prodrugs in accordance with the invention include a compound wherein, one or both hydrogens of the amino functionality of the compound of formula (I) is/are replaced by (C1-C10)alkanoyl.

When the compound of the invention is present in a pharmaceutical composition, it is together with a pharmaceutically acceptable excipient, vehicle or carrier. The term "excipient" is defined as any ingredient other than the compound of formula (I). The choice of excipient will to a large extent depend on factors such as the particular mode of administration, the effect of the excipient on solubility and stability, and the nature of the dosage form. The person skilled in the art is able to formulate a pharmaceutical composition suitable for any given route of administration, e.g. Remington"s Pharmaceutical Sciences, 19th Edition (Mack Publishing Company, 1995).

It is to be understood that all references to "treatment", "treat" or "treating" include curative, palliative and/or prophylactic treatment.

The compound of formula (I) or the pharmaceutical formulations comprising thereof may be preferably administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

Solid formulations for oral administration may be formulated to be immediate and/or modified release.

Modified release formulations include delayed-, sustained-, pulsed-, controlled-, targeted and programmed release.

Further, the compound of formula (I) or the pharmaceutical formulations comprising thereof may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

The person skilled in the art is in the possession all the necessary information to prepare such formulations.

For the purposes of the present invention, especially for the administration to human patients, the total daily dose of the compound of the invention is typically in the range from about any of 10 mg/kg to 25 mg/kg to 50 mg/kg to 100 mg to 150 mg/kg to 200 mg to 250 mg/kg or more, depending, of course, on the mode of administration. For example, the compound of the invention may be administered at about 10 mg/kg, 25 mg/kg, 50 mg/kg, 100 mg, 150 mg/kg, 200 mg or 250 mg/kg.

The total daily dose may be administered in single or divided doses and may, at the physician's discretion, fall outside of the typical range given herein. The preferred dosing regimen is once a day. However, other dosage regimens may be useful, depending on the pattern of pharmacokinetic decay that the physician wishes to achieve. The dosing regimen can vary over time.

These dosages are based on an average human subject having a weight of about 65 kg to 70 kg. The physician will readily be able to determine doses for subjects whose weight falls outside this range.

In another specific embodiment, the compound or composition of the invention is administered chronically. The term "chronic administration" is understood as continuing the dosing regimen for a prolonged time period, such as when the administration lasts for more than three months, preferably more than 6 months, 9 months, a year or more.

The following examples further illustrate the present invention with reference the figures as described below.

### Description of figures

**Fig. 1****.** Original recordings of the surface electrogram and the optical action potentials after perfusion of the heart with 1 µM carbachol.
**Fig. 2****.** Average durations of atrial fibrillation episodes. In the control group, the duration of atrial fibrillation did not decrease for the second trial, while DEA completely prevented the occurrence of atrial fibrillation.

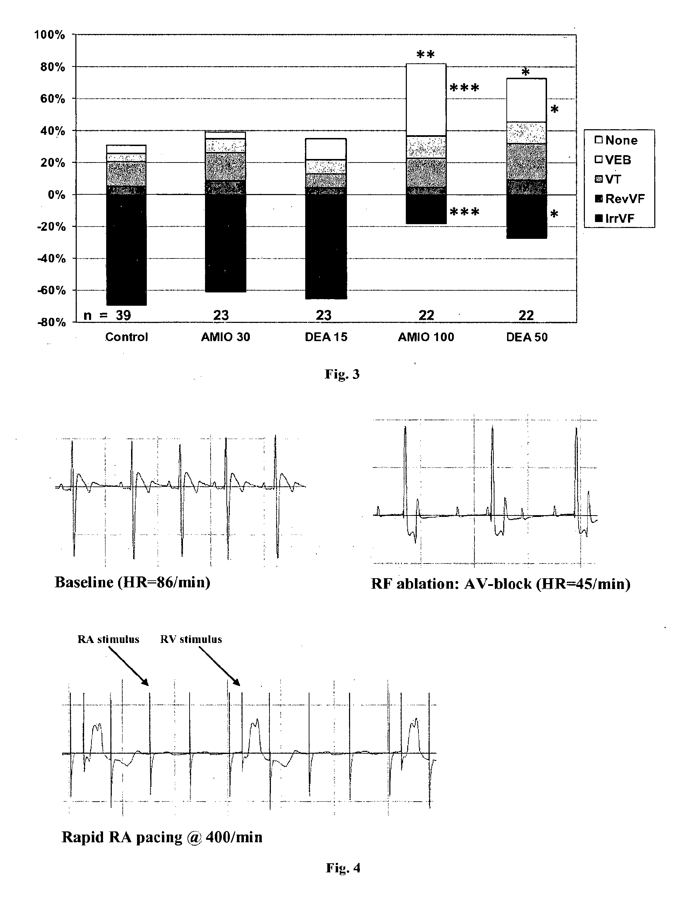

**Fig. 3****.** Influence of 1 month amiodarone (30 mg/kg/d = AMIO 30; 100 mg/kg/d = AMIO 100) or desethylamiodarone (15 mg/kg/d = DEA 15; 50 mg/kg/d = DEA 50) pretreatment on the survival rate and the incidence of arrhythmias during the first 15 min after coronary artery occlusion in conscious rats. IrrVF = irreversible ventricular fibrillation; RevVF = reversible ventricular fibrillation; VT = ventricular tachycardia; VEB = extrasystole, bigeminy, salvo; None = animals that did not develop any arrhythmia. Asterisks denote statistically significant (χ²-probe) difference compared to the control group: * P<0,05 **P<0,01 *** P<0,001.
**Fig. 4****.** Representative ECG recordings before surgery, following AV node ablation and during 400/min right atrial pacing in chronically instrumented dogs. HR=heart rate; RF=radiofrequency; RA= right atrial; RV= right ventricular
**Fig**. **5****.** Representative ECG recordings showing induction of experimental atrial fibrillation using 10-second 800/min frequency burst stimulus in a conscious dog with chronic right atrial pacing induced atrial remodeling. AF=atrial fibrillation.
**Fig. 6****.** Weekly measured plasma levels of desethylamiodarone (µg/ml) during chronic (4-week) oral desethylamiodarone treatment (25 mg/kg/day) in dogs with structural remodelling and atrial fibrillation (n=3). DEA = desethylamiodarone.
**Fig. 7**. **(A)** Weekly measured plasma levels of amiodarone and **(B)** desethylamiodarone levels (µg/ml) during chronic (4-week) oral amiodarone (AMIO) administration (50 mg/kg/day) in dogs with structural remodeling and atrial fibrillation (n=3). AMIO = amiodarone; DEA = desethylamiodarone.
**Fig. 8****.** The effect of chronic (4-week) oral DEA (30 mg/kg/day) and AMIO (45 mg/kg/day) treatment on atrial and ventricular action potential parameters in non-instrumented dogs without structural atrial remodelling.
**Fig. 9****.** The effect of 28-day AMIO and DEA administration on (A) total cholesterol and (B) ALP values in rats. n=3 in each group; **p*<0.05.
**Fig. 10****.** Baseline body weights **(A)** and body weights following 28-day AMIO and DEA administration **(B)** in rats. n=3 in each group; **p*<0.05.
**Fig. 11**. The effect of 28-day AMIO and DEA administration on **(A)** lung weight relative to 100 g body weight, **(B)** on lung weight relative to brain weight, **(C)** on liver weight relative to 100 g body weight and **(D)** on liver weight relative to brain weight in rats. n=7-10 animals/group; **p*<0.05

### Example 1: Desethylamiodarone decreases the incidence of atrial fibrillation in isolated rabbit heart

### Preparation of the isolated heart

Hearts from New Zealand white rabbits (1-2 kg) were used in the experiments. Animals were treated with an intravenous injection of 400 IU/kg heparin and anaesthetized by intravenous infusion of 30 mg/kg pentobarbital and sacrificed by cervical dislocation. The protocols were approved by the Department of Animal Health and Food Control of the Ministry of Agriculture and Rural Development, Hungary (XIII/01031/000/2008) and by the Ethical Committee for the Protection of Animals in Research of the University of Szeged, Szeged, Hungary (approval number I-74-125-2007). After median thoracotomy the heart was quickly removed and placed into cold (4°C) Krebs-Henseleit solution (KHS) containing (in mM): NaCl 118, KC14.3, KH₂PO₄ 1.2, MgSO₄ 1.2, Na pyruvate 5, NaHCO₃ 25, glucose 11, CaCl₂ 1.8, pH 7.4 when gassed with a mixture of 95% O₂ and 5% CO₂. The heart was then mounted on a modified Langendorff apparatus and perfused retrogradely through the aorta with oxygenated KHS warmed to 37°C. The pulmonary vein was also cannulated in order to perfuse the left atrial chamber. In order to record the optical monophasic action potentials the hearts were also loaded with the voltage sensitive fluorescent dye di-4 Anneps for 5 min. To stop the cardiac contractions and avoid motion artefacts during the optical image acquisition the electrical and mechanical activity of the heart was uncoupled by adding 11 mM 2,3-butanedione monoxime to the perfusate.

### Electrophysiological recordings and fluorescence image collection

Epicardial electrograms from the left atrial and left ventricular wall were amplified with a surface electrode amplifier (Experimetria, Hungary) and monitored using a high frequency oscilloscope (Leader Electronics Corporation, Korea). To achieve rapid electrical stimulation (Eltron, Hungary) of the atria custom made electrodes were placed at the top of the anterior part of the vena cava superior. The high resolution optical action potential mapping system consisted of a light-emitting diode (LED) lamp as an excitation light source at a wavelength of 527 nm and a high-resolution, high-speed metal-oxide-semiconductor (CMOS) camera (MiCam02, type MC02C4) equipped with an 580 nm long pass filter for acquiring the fluorescence images from the surface of the heart at a frequency of 833 Hz. Fluorescence images were analyzed using the Brainvison Analyze software (Brainvision Inc Tokyo, Japan).

### Experimental protocol

After allowing the hearts to stabilize for 15 min, acute episodes of atrial fibrillation were induced with rapid electrical stimulation of the atria at a rate of 50Hz for 10 sec in the presence of 1 µM carbachol in the perfusate. The durations of the fibrillation episodes were measured before and after administration of AMIO, DEA or vehicle. All data are expressed as mean ± SEM.

### Drugs

All chemicals were purchased from Sigma-Aldrich (St. Louis, MO, USA), except DEA and di-4 Anneps. Di-4 Anneps was purchased from Molecular Probes Inc. (Eugene, Oregon, USA). DEA was synthesized at the Department of Pharmaceutical Chemistry (Szintekon Kft., Miskolc, Hungary) DEA was dissolved in dimethyl sulfoxide (DMSO) and its final concentrations were 5 µM when diluted in Krebs-Henseleit solution.

### Results

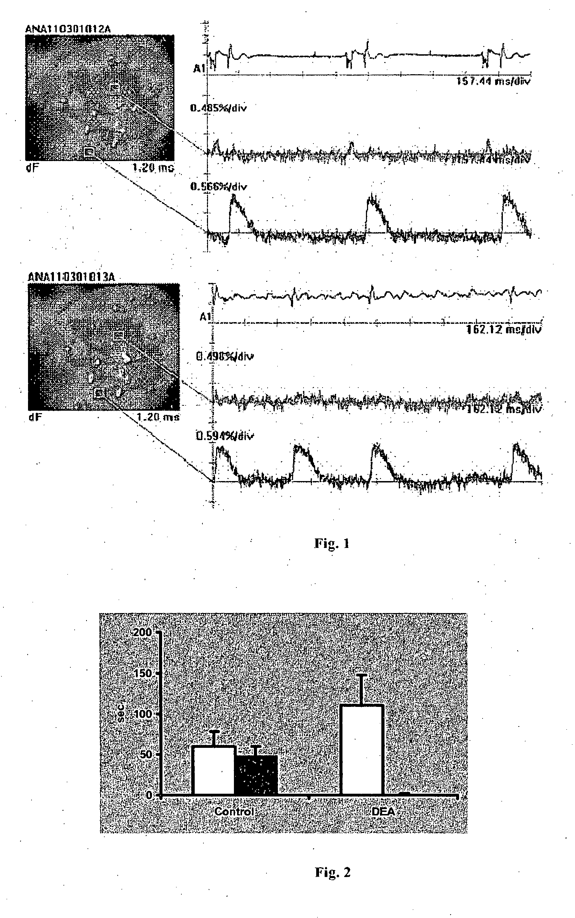

Perfusion of the hearts with 1 µM carbachol markedly slowed down the atrial rhythm thereby sensitizing the atria to fibrillation (Fig. 1.). In baseline conditions with carbachol, in response to a 10 sec rapid atrial pacing atrial fibrillation developed in 13 of 13 hearts in the Control group and in 5 of 5 in the DEA group, showing the validity of our acute atrial fibrillation model. For the second trial of evoking atrial fibrillation, fibrillation occurred in 10 of 13 cases in the Control group. In contrast, perfusion of the hearts with DEA in the DEA group completely prevented the development of atrial fibrillation (0 of 5, Table 1).

**Table 1. Occurrence of atrial fibrillation before and after treatment of the hearts with vehicle or DEA. In the control group, the vehicle alone did not decrease the incidence of fibrillation significantly, while DEA completely prevented the occurrence of fibrillation.**

| | **Before treatment** | **After treatment** | % |
|---|---|---|---|
| **Control** | **13** | **10** | 77 |
| **DEA** | **5** | **0** | **0** |

The average durations of atrial fibrillation episodes in the Control and DEA groups are shown in Table 2 and Fig. 2.

**Table 2. Average durations of atrial fibrillation episodes. In the control group; the duration of fibrillation did not decrease significantly in the second trial, while DEA completely prevented the occurrence of fibrillation.**

| | **Before treatment** | **After treatment** |
|---|---|---|
| **Control** | 59.7±18.3 | 47.9±12.4 |
| **DEA** | 110.2±37.1 | 0±0 |

### Conclusions

These results suggest that DEA may be a promising drug candidate for treatment and/or prevention of atrial fibrillation.

### Example 2: Investigation of the antiarrhythmic effect during acute myocardial infarction in conscious rats

### Coronary artery ligation-induced arrhythmias in conscious rats

The experimental methods used for the investigation of the acute phase of myocardial infarction frequently use anesthetized animals and acute surgical intervention. In such conditions the anesthetic agent, artificial respiration and the acute surgery may greatly and variably influence the events (Baczkó et al., 1997). Therefore, it is especially important to use experimental conditions where the acute phase of myocardial infarction develops in conscious conditions.

The present experiments were performed on male, Sprague-Dawley CFY rats weighing 260-300 g. During a preliminary open-chest surgery we applied a loose silk loop around the left main coronary artery, and then the chest was closed (Leprán et al., 1983). Seven-eight days after the preliminary surgery - after complete recovery and healing - the loose silk loop was tightened to occlude the coronary artery in conscious, freely moving animals. During the first 15 min of myocardial infarction a bipolar ECG was recorded continuously (PowerLab 8SP, ADInstruments, Great Britain).

### Measured parameters

We followed the survival rate during the acute phase (first 15 min) and during the subsequent 16 hours after coronary artery occlusion. The incidence and duration of arrhythmias in the acute phase were evaluated according to the Lambeth Conventions (Walker et al., 1988), i.e. ventricular fibrillation, ventricular tachycardia, and other types of arrhythmias, including ventricular extrasystoles, bigeminy, and salvos. The size of myocardial infarction was measured in the animals surviving for 16 hours after coronary artery occlusion using nitrotetrazolium-blue dye staining.

### Pretreatment

Long-term oral pretreatment was applied for 1 month before the coronary artery occlusion. The applied doses were as follows: AMIO 30 or 100 mg/kg/day (loading dose 100 or 300 mg/kg for 3 days); DEA 15 or 50 mg/kg/day (loading dose 100 or 300 mg/kg for 3 days). Control animals were given the vehicle in a volume of 5 ml/kg.

### Results

Neither AMIO nor DEA produced any behavioral changes of the animals, or in body weight increments. No death occurred due to the 1 month treatment of the animals. Heart rate, measured before the coronary artery occlusion did not differ among different treated groups.

Coronary artery occlusion in conscious rats within 4-6 min resulted in various arrhythmias, leading frequently to irreversible ventricular fibrillation. The incidence of ventricular fibrillation significantly decreased by larger doses of both AMIO and DEA pretreatments (Fig. 3). Both pretreatments significantly improved the survival rate during the acute phase of experimental myocardial infarction. The arrhythmia score, representing the incidence and duration of various arrhythmias and survival as a single number, also significantly decreased (2.05±0.52 and 3.27±0.56 after AMIO 100 and DEA 50 pretreatments, respectively), as compared to the control (4.77±0.33).

At the end of the pretreatments we also determined the concentration of AMIO and DEA in the plasma and the myocardium (Table 3). After AMIO pretreatment its metabolite (i.e. DEA) plasma concentration was about 1/4 of the parent molecule (AMIO). In the myocardium, the tissue concentration of amiodarone was significantly, about 10-times larger, than in the plasma, and the concentration of DEA was equally high. DEA pretreatment produced similar plasma and myocardium concentrations to that measured after amiodarone pretreatment.

**Table 3. Amiodarone (AMIO) and desethyl-amiodarone (DEA) concentration measured in the plasma (PLASMA) or in the myocardium (HEART) after 1 month oral pretreatment.**

| | | **PLASMA** | | **HEART** | |
|---|---|---|---|---|---|
| **Group** | | µ**g/ml** | | **µg/g** | |
| | | **AMIO** | **DEA** | **AMIO** | **DEA** |
| | | | | | |
| **Control** | Mean | 0.00 | 0.00 | 0.00 | 0.00 |
| | SE | 0.00 | 0.00 | 0.00 | 0.00 |
| | n | 4 | 4 | 4 | 4 |
| | | | | | |
| **AMIO** | Mean | 0.68 | 0.15 | 7.91 | 8.95 |
| **100 mg/kg** | SE | 0.10 | 0.03 | 1.25 | 2.21 |
| | n | 12 | 11 | 30 | 30 |
| | | | | | |
| **DEA** | Mean | 0.00 | 0.20 | 0.00 | 7.35 |
| **50 mg/kg** | SE | 0.00 | 0.02 | 0.00 | 0.73 |
| | n | 16 | 16 | 27 | 27 |

In a different group of animals we investigated the possible adverse effects of the long-term pretreatments. For these experiments we used Wistar female rats, known to be more sensitive during toxicological investigations. Amiodarone pretreatment (200 mg/kg/d for 1 month) resulted in a significant decrease in heart rate (376±7.8 vs. 411±14.6 beats/min, n=10), and a prolongation of the PR interval (50±1.3 vs. 46±1.0 msec, n=10) in conscious rats. On the other hand, DEA pretreatment (100 mg/kg/d for 1 month) significantly increased heart rate (437±7.3 beats/min), while the PR interval did not change (45±1.1 msec, n=10), compared to control animals.

### Conclusions

Long-term oral AMIO or DEA pretreatment provided significant protection against life threatening arrhythmias and improved the chance to survive the acute phase of experimental myocardial infarction. This protective effect was produced by similar plasma or myocardial DEA concentrations. However, this effective concentration could be achieved by applying smaller doses of DEA.

### Example 3: Investigation of the antiarrhythmic effect of desethylamiodarone and amiodarone in conscious dogs with chronic rapid atrial pacing induced atrial remodelling and atrial fibrillation

### Animals and surgery

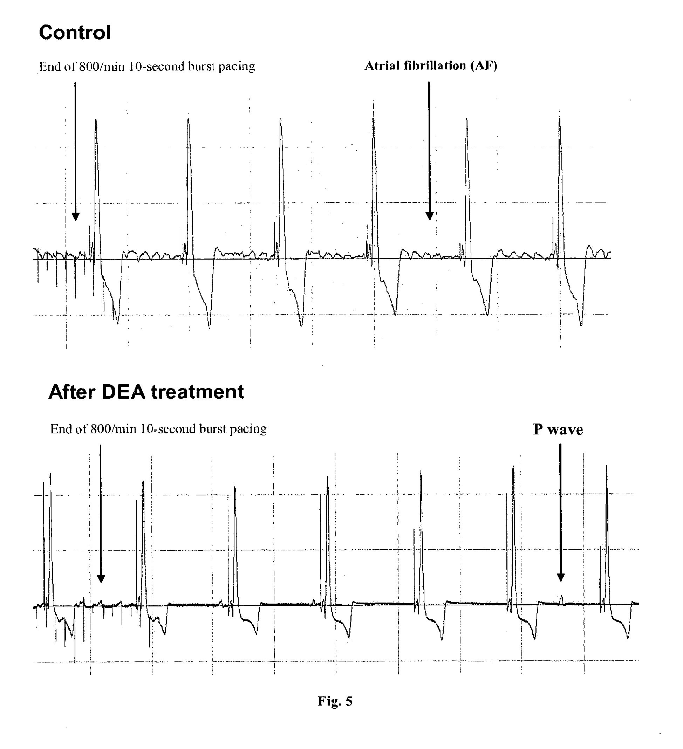

The experiments were performed on chronically instrumented Beagle dogs of both sexes, weighing 12-13 kg. The animals were subjected to the following surgery under general anaesthesia: pacemakers were implanted into bilateral subcutaneous pockets in the neck area (Logos, Karios; Biotronik Hungaria Ltd.) and were attached to pacemaker electrodes implanted into the right ventricle and right atrium. Radiofrequency catheter ablation was performed in each animal to achieve third degree atrioventricular (AV) block so that during subsequent rapid atrial pacing (400/min) the ventricles are protected from high heart rates. The ventricular pacemaker was set to the heart rate to basal heart rate measured before surgery (average 80-90/min). According to our previous experience this heart rate was adequate for routine everyday activities of these animals. On the seventh day after surgery, following the measurement of right atrial effective refractory period the atrial pacemaker was set to a frequency of 400/min to achieve atrial electrical and structural remodelling. Right atrial rapid pacing is necessary to maintain for 3 months in this model to obtain complete remodeling of the atria signalled by the reduction of right atrial effective refractory period below 80 ms. Representative ECG recordings illustrating our dog model are shown on Fig. 4 and Fig. 5.

### Drug administration

Desethylamiodarone was administered in the dose of 25 mg/kg, while amiodarone was administered in the dose of 50 mg/kg (different animals) orally every morning at 7 in previously prepared capsules for 4 weeks. The body weight of animals was monitored for strict adherence to the desired dose.

### Measured parameters

The right atrial effective refractory period (ERP) was measured using the S1-S2 protocol at cycle lengths of 150 and 300 ms. In addition ERP monitoring, 10-second long burst stimuli were applied at 800/min frequency to induce atrial fibrillation and the incidence of AF, the duration of AF episodes were measured before commencement of oral drug therapy and then after the initiation of therapy every 4 days. Blood samples were taken from each animal before treatment and once a week during treatment, the centrifuged plasma was stored at -20°C for later desethylamiodarone and amiodarone level measurements.

### Results

The 4-week oral administration of desethylamiodarone did not cause any visible changes in the mood, behaviour nor did it decrease the body weight of animals.

### Plasma and tissue levels of desetilamiodarone and amiodarone in dogs

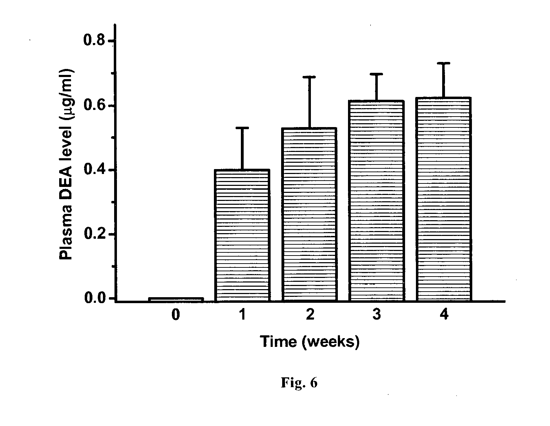

Cardiac tissue drug levels were measured in right atrial, left atrial, right ventricular and left ventricular tissue samples. Following the sacrifice of the animals (the subsequent day after the 4-week treatment), tissue samples were taken before tissue preparations were isolated for *in vitro* studies. The results describing tissue drug levels are summarized in Table 4. It is evident that in all 3 dogs with atrial fibrillation oral treatment was successful yielding appropriate cardiac desethylamiodarone levels. These results are further confirmed by plasma DEA level measurements in these 3 dogs (Fig.6.)

**Table 4. (A) The effect of chronic (4-week) oral desethylamiodarone treatment (25 mg/kg/day) on cardiac tissue desethylamiodarone levels (µg/tissue g); (B) The effect of chronic (4-week) oral amiodarone treatment (50 mg/kg/day) on cardiac tissue amiodarone and (C) desethylamiodarone levels (µg/tissue g) in conscious dogs with atrial fibrillation and structural atrial remodelling.**

| **A DEA treatment** | | | | |
|---|---|---|---|---|
| **Animal** | **Right atrium DEA** | **Left atrium DEA** | **Right ventricle DEA** | **Left ventricle DEA** |
| 2010/06 | 4.763 | 6.683 | 9.652 | 10.368 |
| 2010/11 | 3.296 | 3.827 | 7.544 | 10.568 |
| 2010/14 | 5.743 | 5.795 | 7.116 | 7.972 |
| **Mean** | **4**.**6** | **5**.**4** | **8**.**1** | **9**.**6** |
| **SE** | 0.71 | 0.84 | 0.78 | 0.83 |
| **n** | 3 | 3 | 3 | 3 |

| **B AMIO treatment** | | | | |
|---|---|---|---|---|
| **Animal** | **Right atrium AMIO** | **Left atrium AMIO** | **Right ventricle AMIO** | **Left ventricle AMIO** |
| 2011/1 | 47.005 | 32.632 | 40.787 | 36.603 |
| 2011/8 | 19.936 | 39.045 | 37.033 | 42.573 |
| 2011/9 | 6.933 | 33.89 | 26.349 | 24.061 |
| **Mean** | **24.62** | **35**.**19** | **34**.**72** | **34.41** |
| **SE** | 11.803 | 1.962 | 4.325 | 5.455 |
| **n** | 3 | 3 | 3 | 3 |

| **C AMIO treatment** | | | | |
|---|---|---|---|---|
| **Animal** | **Right atrium DEA** | **Left atrium DEA** | **Right ventricle DEA** | **Left ventricle DEA** |
| 2011/1 | 5.832 | 12.741 | 17.495 | 16.822 |
| 2011/8 | 5.307 | 11.527 | 10.496 | 11.692 |
| 2011/9 | 3.581 | 11.357 | 24.542 | 19.997 |
| **Mean** | **4.91** | **11**.**88** | **17.66** | **16.17** |
| **SE** | 0.680 | 0.436 | 3.926 | 2.419 |
| **n** | 3 | 3 | 3 | 3 |

In the three animals receiving chronic oral amiodarone treatment (50 mg/kg/day) we experienced loss of appetite followed by a reduction in body weight. The first animal lost 4 kgs, the other two 1 kg by the end of the treatment. Loss of appetite and reduction of body weight was not observed with animals treated with desethylamiodarone.

The effects of the 4-week oral desethylamiodarone treatment on the incidence of atrial fibrillation, duration of atrial fibrillation, atrial effective refractory period (ERP) in conscious dogs with atrial structural remodelling are summarized in Table 5. In three animals, the incidence of atrial fibrillation, the duration of atrial fibrillation markedly and significantly decreased accompanied by the prolongation of the ERP.

**Table 5. The effect of chronic (4-week) oral desethylamiodarone (DEA) treatment (25 mg/kg/day) on the incidence and duration of burst-induced atrial fibrillation and on atrial effective refractory period (ERP; ms) in conscious dogs with structural atrial remodelling. AF=atrial fibrillation.**

| **DEA** | **Control** | | | | **Following 4-week treatment** | | | |
|---|---|---|---|---|---|---|---|---|
| **Animal** | **ERP** | **Incidence of AF** | **AF duration (s)** | **Lg AF duration** | **ERP** | **Incidence of AF** | **AF duration (s)** | **Lg AF duration** |
| 2010/06 | <80 | 60% | 1 122.6 | 3.05 | 80 | 10% | 1.9 | 0.28 |
| 2010/11 | <80 | 50% | 1 505.8 | 3.17 | 80 | 10% | 21.6 | 1.33 |
| 2010/14 | <80 | 27% | 6739.1 | 3.82 | <80 | 27% | 44 | 1.64 |
| **Mean** ± **SE** | | 45.7 ± 9.77 | 3122 ± 1811.7 | 3.35 ± 0.24 | | 15.7 ± 5.67 | 22.5 ± 12.16 | 1.08 ± 0.41* |

The effects of 4-week oral amiodarone treatment on incidence of atrial fibrillation, duration of atrial fibrillation and effective refractory period (ERP) in conscious dogs with structural atrial remodelling are summarized in Table 6. In three animals, the incidence and duration of atrial fibrillation showed a decreasing tendency accompanied by the prolongation of the ERP.

**Table 6. The effect of chronic (4-week) oral amiodarone (AMIO) treatment (50 mg/kg/day) on the incidence and duration of burst-induced atrial fibrillation and on atrial effective refractory period (ERP; ms) in conscious dogs with structural atrial remodelling. AF=atrial fibrillation. *p<0.05**

| **AMIO** | **Control** | | | | **Following 4-week treatment** | | | |
|---|---|---|---|---|---|---|---|---|
| **Animal** | **ERP** | **Incidence of AF** | **AF duration (s)** | **Lg AF duration** | **ERP** | **Incidence of AF** | **AF duration (s)** | **Lg AF duration** |
| 2011/01 | 90 | 100% | 386.5 | 2.59 | 100 | 40% | 22.5 | 1.35 |
| 2011/08 | <80 | 90% | 1 302.9 | 3.12 | 130 | 10% | 7.5 | 0.88 |
| 2011/9 | 80 | 67% | 22 663.7 | 4.35 | 100 | 10% | 1.31 | 0.12 |
| **Mean** ± **SE** | | 88.0 ± 10.54 | 8117.7 ± 7277.8 | 3.35 ± 0.52 | | 20.0 ± 10.0* | 10.4 ± 6.29 | 0.78 ± 0.36* |

In summary, chronic oral amiodarone (50 mg/kg/day) and desethylamiodarone (25 mg/kg/day) treatment effectively and similarly decreased the incidence of atrial fibrillation, the duration of atrial fibrillation episodes and increased atrial effective refractory periods in conscious, chronically instrumented Beagle dogs.

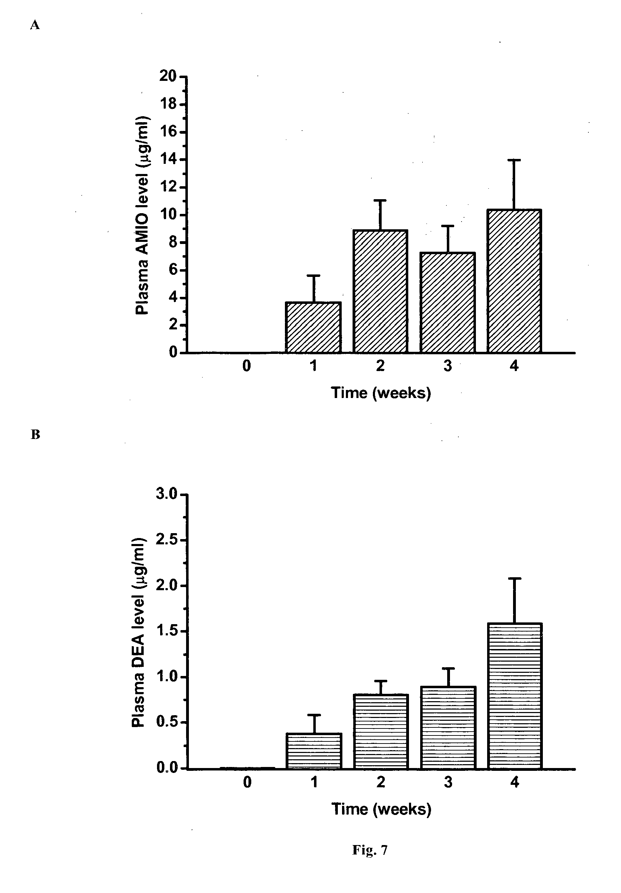

**The two drug treatments yielded markedly different plasma (****FIGURES 6-7****), but** similar cardiac tissue desethylamiodarone levels (Table 4). On the other hand, as shown in Table 7, liver and lung tissue DEA levels were significantly and markedly higher following AMIO treatment than following DEA treatment. These results are in good agreement with results showing DEA accumulation in human alveolar epithelium-derived cell lines following AMIO treatment (Seki *et al*., 2008). These results suggest that similar therapeutic antiarrhythmic effects are associated with similar cardiac tissue concentrations following AMIO and DEA treatments, however, they result in strikingly different liver and lung tissue concentrations of DEA and AMIO. Based on this observation it can be concluded that chronic AMIO treatment would greatly enhance the risk for hepato- and pulmonary toxic complications compared to treatment with DEA alone.

**Table 7. The effect of chronic (4-week) oral DEA (25 mg/kg/day) and AMIO (50 mg/kg/day) treatment on liver and lung tissue DEA and AMIO levels µg/tissue g) in dogs with structural atrial remodelling. *p<0.05**

| **DEA treatment** | | | | |
|---|---|---|---|---|
| **Animal** | **Liver DEA** | **Lung DEA** | | |
| 2010/6 | 9.609 | 69.977 | | |
| 2010/11 | 9.9128 | 34.912 | | |
| 2010/14 | 32.801 | 50.029 | | |
| **Mean** ± **SE** | 17.4 ± 7.68 | 51.6 ± 10.15 | | |

| **AMIO treatment** | | | | |
|---|---|---|---|---|
| **Animal** | **Liver DEA** | **Lung DEA** | **Liver AMIO** | **Lung AMIO** |
| 2011/1 | 119.504 | 362.062 | 123.961 | 312.24 |
| 2011/8 | 114.865 | 168.064 | 200.27 | 227.793 |
| 2011/9 | 54.424 | 153.058 | 56.314 | 105.152 |
| **Mean** ± **SE** | 96.3 ± 20.96* | 227.7 ± 67.31 | 126.8 ± 41.58 | 215.1 ± 60.12 |

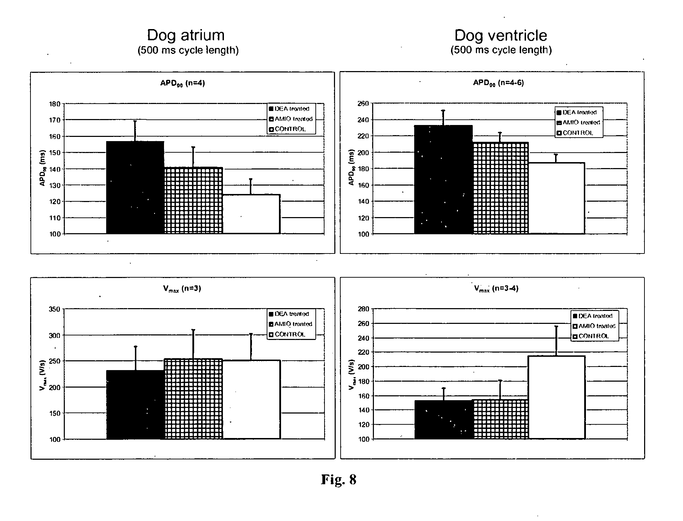

In additional chronic experiments on non-instrumented Beagle dogs, two animals were treated with amiodarone 45 mg/kg/day and two animals with desethylamiodarone 30 mg/kg/day orally, for 4 weeks. As Table 8 and Fig. 8 show, chronic desethylamiodarone (metabolite) treatment elicited similar or even more marked cardiac electrophysiological changes, i.e. lengthening of the atrial and ventricular action potential duration (APD) defined as Class III antiarrhythmic property, and decreased the maximal rate of depolarization (Vₘₐₓ), defined as Class I antiarrhythmic mechanism compared to the parent compound amiodarone.

**Table 8. The effect of chronic (4-week) oral DEA (30 mg/kg/day) and AMIO (45 mg/kg/day) treatment on atrial and ventricular action potential parameters in non-instrumented dogs without structural atrial remodelling.**

| **Dog atrium** | | | | **Dog ventricle** | | |
|---|---|---|---|---|---|---|
| (500 ms cycle length) | | | | (500 ms cycle length) | | |
| | APD₉₀ (ms) | Vₘₐₓ (V/s) | | | APD₉₀ (ms) | Vₘₐₓ(V/s) |
| | (n=4) | (n=3) | | | (n=4-6) | (n=3-4) |
| DEA treated | 156.6±12.6 | 231.5±45.4 | | DEA treated | 232.5±18.3 | 153.0±17.2 |
| AMIO treated | 140.8±12.4 | 252.9±56.7 | | AMIO treated | 211.4±12.7 | 153.8±27.4 |
| CONTROL | 123.9±9.6 | 249.7±52.7 | | CONTROL | 186.3±10.8 | 214.8±40.9 |

The corresponding plasma and tissue levels from these dogs are summarized in Table 9.

**Table 9. The effect of chronic (4-week) oral DEA (30 mglkg/day) and AMIO (45 mg/kg/day) treatment on plasma, cardiac, liver and lung tissue DEA and AMIO levels (µg/tissue g) in non-instrumented dogs without structural atrial remodeling.**

| **DEA levels following DEA and AMIO treatments** | | | | | | |
|---|---|---|---|---|---|---|
| **Animal** | **Plasma** | **Right Atrium** | **Left Ventricle** | **Liver** | **Lung** | **Kidney** |
| DEA dog 1 | 0.397 | 15.522 | 35.215 | 94.864 | 58.164 | 26.887 |
| DEA dog 2 | 0.505 | 19.866 | 39.939 | 161.290 | 127.432 | 53.615 |
| *AMIO dog 1* | 0.531 | 19.768 | 47.670 | 63.399 | 156.841 | 65.429 |
| *AMIO dog 2* | 0.594 | 30.071 | 51.173 | 132.501 | 244.176 | 74.371 |

| **AMIO levels following AMIO treatment** | | | | | | |
|---|---|---|---|---|---|---|
| **Animal** | **Plasma** | **Right Atrium** | **Left Ventricle** | **Liver** | **Lung** | **Kidney** |
| *AMIO dog 1* | 7.048 | 75.854 | 94.348 | 114.066 | 158.583 | 113.987 |
| *AMIO dog 2* | 4.757 | 152.023 | 81.928 | 177.317 | 178.360 | 112.173 |

### Conclusions

These new and unpublished experimental data show that chronic treatment with 30 mg/kg desethylamiodarone resulted in similar antiarrhythmic cellular electrophysiological changes in cardiac atrial and ventricular tissue to 45 mg/kg chronic amiodarone treatment. The tissue levels for desethylamiodarone both in the cardiac atrial and ventricular tissue were similar. The same seems to be true for liver, lung and kidney tissue levels which can be important for possible organ toxicity issues. It is important to emphasize that during chronic amiodarone treatment in addition to the metabolite (desethylamiodarone) deposition even higher tissue amiodarone depositions were observed in the heart, lung, liver and the kidney. Since there are no amiodarone tissue depositions after chronic desethylamiodarone treatment it can be assumed that following chronic desethylamiodarone treatment similar therapeutic cardiac electrophysiological effects can be excepted as with the treatment with the parent compound (amiodarone) alone, however, the organ toxicity in lung, liver and kidney would be more pronounced after chronic amiodarone compared to chronic desethylamiodarone treatment. This latter should argue for the better therapeutic value of desethylamiodarone compared to that of amiodarone. Also, the markedly lower plasma drug concentrations after chronic desethylamiodarone treatment should cause fewer possible pharmacokinetic interactions with other drugs than that following chronic amiodarone treatment.

### Example 4: Summary of chronic, 28-day toxicology results in non-GLP rats

The results of preliminary non-GLP 28-day toxicology investigations suggest that oral 200 mg/kg/day treatment with amiodarone (AMIO; n=9) yielded markedly different results versus animals treated with vehicle (n=10) in comparison to animals receiving 100 mg/kg/day desethylamiodarone (DEA; n=7). The dose selection was based on the finding that in previous efficacy studies in rats the dose of AMIO needed to achieve certain cardiac tissue levels of DEA that exerted a similar antiarrhythmic effect (8.9±2.1 µg/g in left myocardium, n=30; 6.8±1.9 µg/g in right myocardium, n=24) was twice as high (100 mg/kg/day) compared to the required DEA dose (50 mg/kg/day). Following 21-day oral DEA administration 7.3±0.7 µg/g (n=27) DEA tissue level was measured in the left ventricular myocardium and 8.6±1.1 µg/g (n=16) DEA concentration was detected in the right ventricular myocardium.

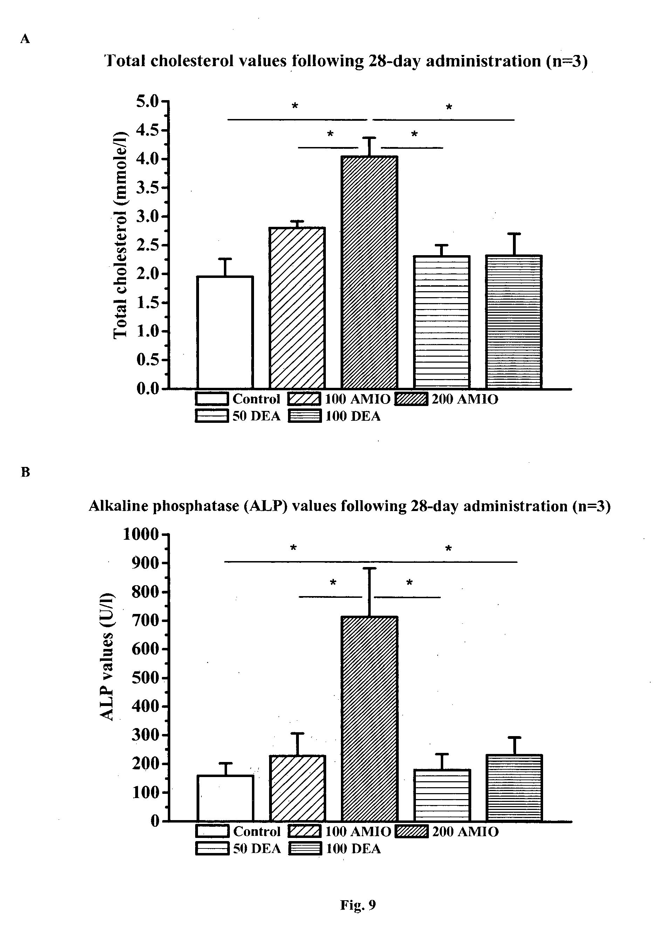

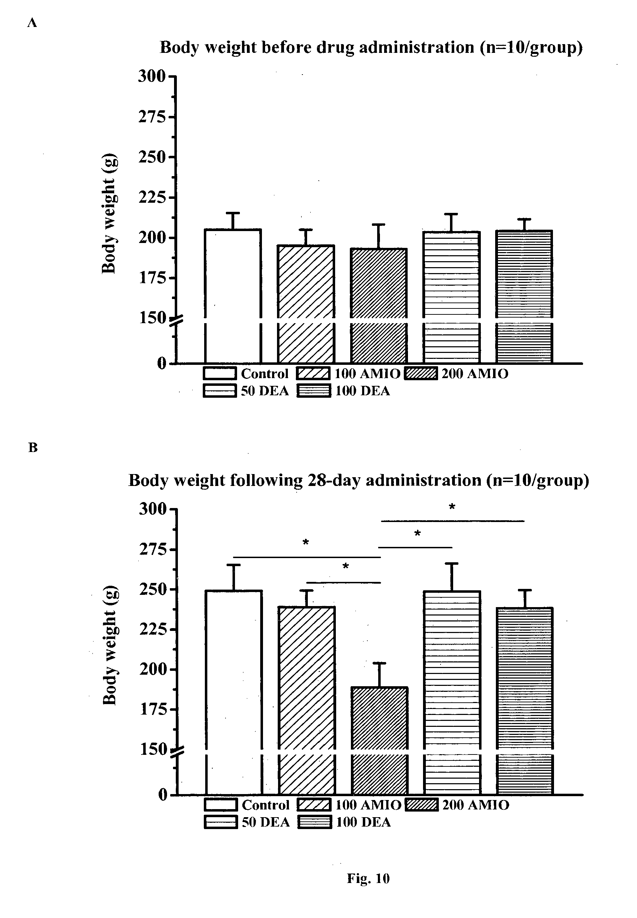

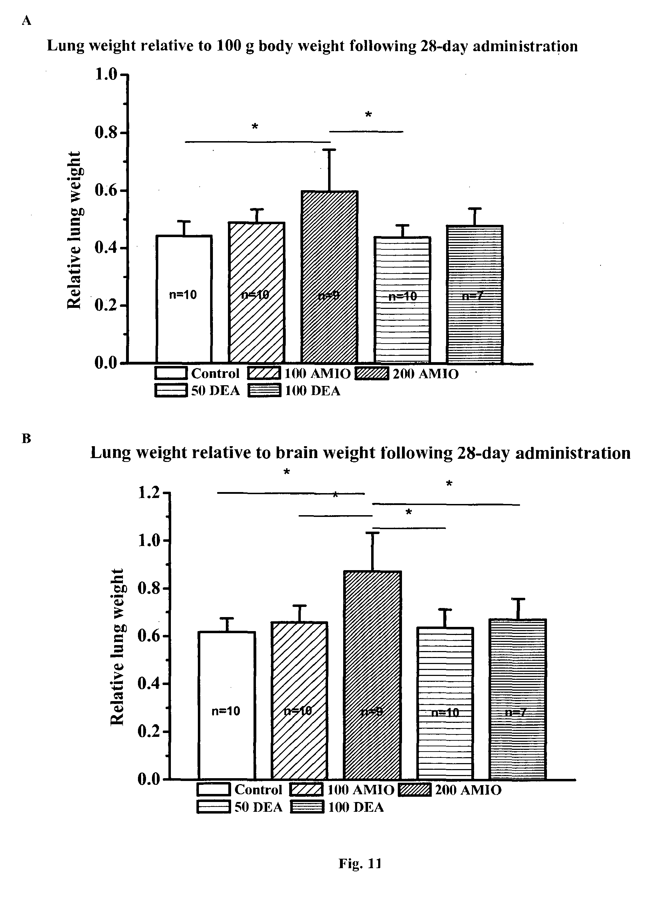

The most important differences following DEA and AMIO administration can be summarized as follows:
1. 14 days after the completion of 200 mg/kg/day AMIO administration AMIO could be detected in plasma samples, while following 100 mg/kg/day DEA administration DEA was not detected in plasma or cardiac tissue samples. These results suggest that the elimination of DEA is faster than that of AMIO - a favourable pharmacokinetic feature since the accumulation and toxic adverse effects of the drug are reduced during chronic drug administration.
2. As Fig. 9 illustrates, hepatic function alterations in animals treated with 200mg/kg/day AMIO were more robust compared to those animals treated with 100 mg/kg/day DEA (total cholesterol, alkaline phosphatase [ALP]). These results may suggest that treatment with the metabolite (DEA) leads to reduced hepatotoxic side effects compared to treatment with amiodarone (AMIO).
3. In the lungs, following 200 mg/kg/day AMIO treatment alveolar histiocytosis was detected while after 100 mg/kg/day DEA treatment no such observation was made. This pathological finding may suggest that DEA treatment might be more beneficial considering one of the most serious adverse effects of chronic AMIO treatment, the development of pulmonary fibrosis.
4. As shown on Fig 10., following 28-day treatment with 200 mg/kg/day AMIO, the normal increase in body weight (44 g) was absent (13 g). Following 100 mg/kg/day DEA treatment body weight reduction was not observed (34 g). This result may be attributed to the observation that during the first 3 weeks of 200 mg/kg/day AMIO treatment food consumption and appetite of the animals were reduced compared to control and 100 mg/kg/day DEA treated animals.
5. Following treatment with 200 mg/kg/day AMIO, the weight of the liver was significantly increased both when normalized to body weight and to brain weight. These respective measurements yielded no significant differences after 100 mg/kg/day DEA treatment (Fig. 11). These results suggest increased hepatotoxic and pulmonary toxic adverse effects following AMIO treatment as opposed to treatment with the metabolite, DEA.

Our own experimental results that are unique in the scientific literature show for the first time that in order to achieve similar antiarrhythmic effects both against atrial fibrillation and ventricular arrhythmias in rats, rabbits and dogs, half the dose of DEA needs to be administered than AMIO. These effects are accompanied by similar cardiac tissue DEA levels after both DEA and AMIO (twice higher dose) treatments. Importantly, according to chronic toxicological studies in rats following 28-day oral treatments, DEA (50 mg/kg/day) administration in half the dose of AMIO (100 mg/kg/day) administration led to reduced pathological alterations in the lungs and the liver, i.e. smaller doses of DEA exert similar antiarrhythmic effects while causing milder toxic and adverse effects.

### References

Baczkó I, Leprán I, Papp JGy: Influence of anaesthetics on sudden death and the incidence and duration of reperfusion-induced arrhythmias in rats. J. Cardiovasc. Pharmacol. 29: 196-201. 1997.
Bolderman RW, et al: "Atrium-targeted drug delivery through an amiodarone-eluting bilayered palch", Journal Of Thoracic And Cardiovascular Surgery, Mosby-Year Book, Inc., St. Louis, MO, US, vol. 140, no. 4,1 October 2010
Flanagan RJ, Storey GC, Holt DW, Farmer PB. Identification and measurement of desethymamiodarone in blood plasma specimens from amiodarone-treated patients. J. Pharm. Pharmacol. 34: 638-643, 1982.
Guang-Fei Shi ET AL: "Mechanism of the delayed onset of action after intravenous amiodarone for treatment of ventricular arrhythmias". Drug development research, vol. 58, no. 1, 1 January 2003
Jahanavi Kharidia et al: "Effects of desethylamiodarone on the electrocardiogram in conscious freely moving animals: pharmacokinetic and pharmacodynamic modeling using computer-assisted radio telemetry". Biopharmaceutics & Drug Disposition, vol. 17, no. 2,1 March 1996 (1996-03-01), pales 93-106
Kato R, Van Ketesh N, Kamiya K, Yabek S, Kannan R, Singh BN. Electrophysiologic effects of desethylamiodarone, an active metabolite of amiodarone: comparison with amiodarone during chronic administration in rabbits. Am. Heart J. 9:106.9; 1988.
Latham KR, Sellitti DF, Goldstein RE: Interaction of amiodarone and desethylamiodarone with solubolozed nuclear thyroid hormone receptors. J Am Cardiol. 9:972-876; 1987.
Leprán I, Koltai M, Siegmund W, Szekeres L: Coronary artery ligation, early arrhythmias, and determination of the ischemic area in conscious rats. J. Pharmacol. Meth. 9: 219-230. 1983.
Nattel S, Davies M, Quantz M. The antiarrhythmic effect of amiodarone and desethylamiodarone, alone and in combination, in dog with acute myocardiac infarction. Circulation. 77:200-8; 1988.
Nattel S: Pharmacodynamic Studies of Amiodarone and Its Active N-Desetyl Metabolite. J. Cardiocvasc. Pharmacol. 8:771-777; 1986.
Ravens U: Antiarrhythmic therapy in atrial fibrillation. Pharmacol. & Ther. 128: 129-145, 2010.
Seki S, Itagaki S, Kobayashi M, Hirano T, Iseki K. Amiodarone Increases the Accumulation of DEA in a Human Alveolar Epithelium-Derived Cell Line. Biol. Pharm. Bull. 31:1449-52; 2008.
Shi RO, Lee JK, Hayashi Y, Takeuchi Y, Kambe F, Futaki S, Seo H, Murata Y, Kodama I. Long-term amiodarone treatment causes cardioselective hypothyroid-like alteration in gene expression profile. Eur J Pharmacol. 578:270-8; 2008.
Shinagawa K, Shiroshita-Takeshita A, Schram G, Nattel S. Effects of antiarrhythmic drugs on fibrillation in the remodelled atrium: insights into the mechanism of the superior efficacy of amiodarone. Circulation. 107:1440-6; 2003.
Stark G. et al: "Comparison of acute electrophysiological effects of amiodarone and its metabolite desethylamiodarone in Langendorff perfused guinea pig hearts", Basic Research In Cardiology, Steinkonff, Darmstadt, DE, vol. 86, no. 2,1 March 1991
Talajic M. De Roode MR, Nattel S. Comparative electrophysiologic effects of intravenous amiodarone and desethylamiodaropne in dogs: evidence for clinically relevant activity of the metabolite. Circulation. 75:265-71; 1987.
Tieleman RG, et al.: "Efficacy, Safety, and Determinants of Conversion of Atrial Fibrillation and Flutter With Oral Amiodarone", The American Journal Of Cardiology, vol. 79, No. 1, 1 January 1997
Tisdale JE, Follin SL, Ordelova A, Webb CR: Risk factors for the development of specific noncardiovascular adverse effects associated with amiodarone. J. Clin. Pharmacol. 35: 351-356, 1995.
van Beeren HC, Bakker O, Wiersinga WM. Desthylamiodarone is a competitive inhibitor of the binding of thyroid hormone to the thyroid hormone alpha 1-receptor protein. Mol Cell Endocrinol. 112:15-9; 1995.
van Beeren HC, BakkerO, Wiereinga WM. Desethylamiodarone interferes with the binding of co-activator GRIP-1 to beta 1-thyroid hormone receptor. FEBS Lett. 450:35-8; 1999.
Varró A, Bódi I, Rabloczky G: Antiarrhythmic effect of desethylamiodarone in conscious rats. J. Pharm. Pharmacol. 39:483-484; 1987.
Walker MJA, Curtis MJ, Hearse DJ, Campbell RWF, Janse MJ, Yellon DM, Cobbe SM, Coker SJ, Harness JB, Harron DWG, Higgins AJ, Julian DG, Lab MJ, Manning AS, Northover BJ, Parratt JR, Riemersma RA, Riva E, Russell DC, Sheridan DJ, Winslow E, Woodward B: The Lambeth Conventions: guidelines for the study of arrhythmias in ischemia, infarction, and reperfusion. Cardiovasc. Res. 22: 447-455; 1988.
Wang Z, Feng J, Shi H, Pond A, Nerbonne JM, and Nattel S. Potential molecular basis of different physiological properties of the transient outward K+ current in rabbit and human atrial myocytes. Circ Res 84: 551-561; 1999.
Wang Z, Fermini B, Feng J, Nattel S. Role of chloride currents in repolarizing rabbit atrial myocytes. Am J Physiol 268(5 Pt 2): H1992-H2002; 1995.
Zhou L, Chen BP, Kluger J, Fan C, Chow MS. Effects of amiodarone and its active metabolite desethylamiodarone on the ventricular defibrillation threshold. J Am Coll Cardiol. 31 (7):1672-1678; 1998.
Zhou L, et al: "A comparison of the antifibrillatory effects of desethylamiodarone to amiodarone in a swine model", Journal Of Cardiovascular Pharmacology, Raven Press, New York, NY, vol. 34, no. 3, 1 January 1999

## Claims

1. A pharmaceutical composition comprising a compound selected from the group consisting of desethylamiodarone and pharmaceutically acceptable salts, hydrates and solvates thereof, together with a pharmaceutically acceptable excipient, vehicle and/or carrier, for use in the treatment and/or prevention of atrial fibrillation by oral administration.

2. The composition for use according to claim 1, wherein the compound or composition is administered orally, sublingually, or buccally.

3. The composition for use according to claim 1 or 2, wherein the composition is administered chronically.

4. The composition for use according to any one of claims 1 to 3, wherein the composition is administered once a day.

## Patentansprüche

1. Eine Arzneimittelzusammensetzung enthaltend eine Verbindung ausgewählt aus der Gruppe enthaltend Desethylamiodarone sowie dessen pharmazeutisch annehmbare Salze, Hydrate und Solvate, zusammen mit pharmazeutisch annehmbaren Hilfsstoffen und/oder Trägerstoffen, zur Anwendung in der Behandlung und/oder Prävention von Vorhofflimmern mit oraler Verabreichung.

2. Die Zusammensetzung zur Anwendung nach Anspruch 1, wobei die Verbindung oder die Zusammensetzung oral, sublingual oder buccal verabreicht wird.

3. Die Zusammensetzung zur Anwendung nach Anspruch 1 oder 2, wobei die Zusammensetzung kontinuierlich verabreicht wird.

4. Die Zusammensetzung zur Anwendung nach einem der Ansprüchen 1 bis 3, wobei die Zusammensetzung einmal täglich verabreicht wird.

## Revendications

1. Une composition pharmaceutique comprenant un composé choisi dans le groupe constitué de déséthylamiodarone et de ses sels, hydrates et / ou solvates pharmaceutiquement acceptables, conjointement avec un excipient, véhicule et / ou support pharmaceutiquement acceptable, pour l'utilisation dans le traitement ou la prévention de la fibrillation auriculaire par l'administration par voie orale.

2. Composition pour l'utilisation selon la revendication 1, dans laquelle le composé ou la composition est administré(e) par voie orale, sublinguale ou buccale.

3. Composition pour l'utilisation selon la revendication 1 ou 2, dans laquelle la composition est administrée de manière chronique.

4. Composition pour l'utilisation selon l'une quelconque des revendications 1 à 3, dans laquelle la composition est administrée une fois par jour.
